# EUROPEAN PATENT SPECIFICATION

(11) **EP 2 851 015 B1**
(45) Date of publication and mention of the grant of the patent: **08.11.2017**
(21) Application number: 13425125.5
(22) Date of filing: 20.09.2013
(51) Int. Cl.: A61B 17/12

(54) **Temporary venous transfemoral endoprosthesis for a total vascular exclusion of the liver**
Vorübergehende venöse transfemorale Endoprothese für eine totale vaskuläre Exklusion der Leber
Endoprothèse transfémorale veineuse pour une exclusion vasculaire temporaire et totale du foie

(43) Date of publication of application: 25.03.2015
(73) Proprietor: Truosolo, Bruno Alberto Vittorio, 00124 Roma (IT)
(72) Inventor: Truosolo, Bruno Alberto Vittorio, 00124 Roma (IT)
(74) Representative: Zizzari, Massimo

(56) References cited:
- EP-A1- 2 596 828
- WO-A1-00/12169
- WO-A1-99/48545
- WO-A2-2008/091991
- US-A- 5 257 975
- US-A- 6 162 237

## Description

The present invention regards a medical device that is represented by a temporary venous transfemoral or trans-saphena endoprosthesis for a total vascular exclusion of the liver. This device results to be particularly adapt to be used in most critical surgical operations involving this organ, like in example in *major hepatectomy* and in *hepatic trauma* with caval upper hepatic venous damage.

In fact, the endoprosthesis represents an internal and temporary endovenous bypass that permits the normal function of the caval vein to return the blood to heart, and at the same time permits, with a simultaneous *Pringle maneuver,* the total vascular exclusion of the hepatic parenchyma.

The liver receives blood coming from the portal vein and from the hepatic vein, and returns filtered blood to the caval system through the three upper hepatic veins.

The vascular exclusion of the liver, according to the invention, consists in stopping the blood entering the liver, by blocking the hepatic peduncle (hepatic artery, common bile duct and portal vein) with a clamp or a tourniquet, and stopping simultaneously the way out, in such a way that the blood cannot enter the liver from the inferior caval vein through the upper hepatic veins.

Therefore, it is used the *Pringle maneuver,* that is well known from the current surgical techniques, in order to stop the way of the blood to the liver, and it is blocked the way out of the liver closing the upper hepatic veins, by intervention directly on the caval vein, from the inside. This blocking of the way out is achieved by installation of an endoprosthesis, that is inserted directly from the femoral vein or saphena and then it is pushed inside the inferior caval vein, reaching with the distal part the caval lower and upper hepatic tract, blocking therefore with the lateral walls the holes connecting the upper hepatic veins to the caval vein.

The endoprosthesis is composed of a main part having a cylindrical shape extending longitudinally. The part that, once it has been pushed in the caval vein, is placed close to the distal part of a inner guide, opens a spiral shaped self-expandable thin sheet, in order to bond steadily to the inside wall of the caval vein. The expansion saves an inner caval space that is necessary to keep the flow of blood inside, so that the function of blood return to heart is saved.

From the prior art some attempts are known in order to solve the problem of total vascular exclusion of the liver.

A first technique, known as *quadruple clamping,* consists in using the *Pringle maneuver* in order to stop the way of the blood to the liver, clamping the aorta under the diaphragm, and clamping the caval vein in the upper and lower hepatic tract. This complicated technique (often the clamping point of the upper hepatic tract in the caval vein is in the preatrial tract) could give serious damage when the clamping is removed, causing a shock that sometimes could lead to death.

Another solution, very difficult or even impossible achieve in a condition of emergency, is to provide an extracorporeal circulation as defined in the protocols of liver transplantation.

The solution disclosed In patent US 6.162.237 describes instead an endoprosthesis that is inserted in the inferior caval vein. It presents a distal part having a structure that could be extended radially. The structure is composed of supporting elements that are connected at one end to an inner guiding wire, and at the other end to an outer guiding wire. After the positioning of the device, with the distal part in the hepatic tract of the caval vein, the motion of the outer guiding wire in respect to the inner one, causes a radial opening of the supporting elements following an umbrella-like mechanism. In such a way, the distal part extends itself radially until it bonds to the inside wall of the caval vein, blocking therefore with the lateral walls the holes connecting the upper hepatic veins to the caval vein. This patent also discloses extension or retraction of a sheath upon selective rotation of a drive wire. When the structure extends itself out, being open in the inner side, permits the inner flow of blood, keeping therefore the normal circulatory function of the patient.

This solution presents the serious drawback of a surgeon that cannot have the sensation of the level of opening of the device, leading to a risk of damage or making worse the situation of wounds in the caval upper hepatic circulation area. Furthermore, a not perfect and weak bond to the inner wall makes this solution not very effective in respect of the goal of closing the holes connecting the upper hepatic veins to the caval vein.

The device disclosed in patent US 4.950.226 represents an endoprosthesis that is also inserted in the inferior caval vein. It is composed of two coaxial cylinders, where the first is internal and can translate in respect to the other that is external. Once the prosthesis has been placed with the distal part in the hepatic tract of the caval vein, two balloons are inflated respectively in the upper and lower side in respect to the caval hepatic tract, in order to isolate the upper hepatic veins in respect to the circulation of blood.

This solution presents many drawbacks. The first is to represent a particularly large device that requires a complicated insertion from the external iliac vein. Furthermore, also in this case a surgeon cannot have the sensation of the level of opening of the device, leading to a risk of damage or making worse the situation of wounds in the caval upper hepatic circulation area.

Therefore, the subject of the present invention is given by an endoprosthesis for a total vascular exclusion of the liver, overcoming all the above drawbacks, permitting a comfortable use because can be applied and adapted to different patients having different size and extension of the inner anatomical structures.

In particular, the endoprosthesis permits to keep the normal function of the caval vein to return the blood to heart, all during the time of the surgical intervention, avoiding a partial or total block of the circulation, and avoiding the possibility to a temporary extracorporeal circulation.

Furthermore, the present invention permits to achieve a highly precise control of the level of expansion of the prosthesis, a better perception by the surgeon of the reached level of expansion, a better adherence to the inner walls of the caval vein and an easier procedure if insertion and installation of the same prosthesis.

Therefore, it is specific subject of the present invention a venous transfemoral endoprosthesis for a total vascular exclusion of the liver, to be used in most critical surgical operations involving the hepatic parenchyma, like in example in *major hepatectomy* and in *hepatic trauma* with caval upper hepatic venous damage, characterized in that comprising:
- an endovenous catheter, with a millimetric scale, having the shape of a cylinder extended longitudinally, radiopaque and flexible in order to be inserted from the femoral vein or the saphena vein; the endovenous catheter includes a protected input/output door, that is a secondary channel used for washing;
- a semi-rigid guiding element, internal to the endovenous catheter, having a distal rounded and radiopaque head (the radiopaque landmarks permit the positioning directly under a CT-scan before the intervention; the millimetric scale of the external catheter permits the positioning in the hospital according to the tomographic data or, in case of very critical patient conditions from a hemodynamic point of view, directly in the surgical room, after the *Pringle maneuver* and the manual positioning of the perihepatic radiopaque landmarks);
- a thin sheet (having memory of shape or being expandable with heat) fixed and rolled up on the distal part of said guiding element; the lower side of the sheet, near the main catheter, is oblique and has a rounded border; the proximal part of the guiding element includes a screw mechanism that extends itself outside the main venous catheter; once the position of the venous catheter is fixed, some small lateral walls having holes for fixing by stitches permit the stable installation of the device,
   so that the screwing motion given to the guiding element from the proximal part (piston placed outside the main catheter having a proper thread and a counter-thread in the main endovenous catheter) permits to retract the main venous catheter in order to free the distal self expandable sheet that tends to expand adapting itself perfectly to the caval surface, keeping the normal function of venous blood return to heart; the opposite motion of the piston retracts the thin sheet that has a squared shape properly designed to favorite the retraction of the main distal catheter.

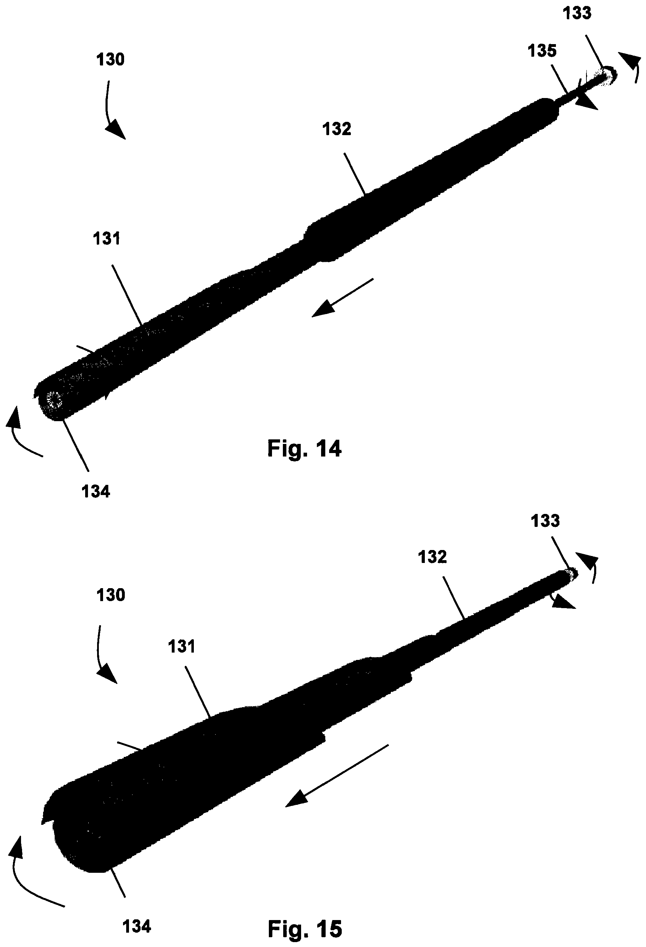

The present invention will now be described for illustrative but not limitative purposes, with particular reference to figures 14 and 15 of the enclosed drawings. Embodiments of medical devices for total vascular exclusion of the liver are also disclosed in the enclosed drawings, wherein:
Figure 1 is a lateral view of a temporary venous transfemoral or trans-saphena endoprosthesis for a total vascular exclusion of the liver, according to a first embodiment called *"by expansion"*;
Figure 2 is a perspective lateral view of the same endoprosthesis of Figure 1;
Figure 3 is a lateral view of a particular of the front part surface of the same endoprosthesis of Figure 1, wherein a mechanism of wire traction defines the radial compression of the same surface;
Figure 4 is a lateral view of a particular of the front part surface of the same endoprosthesis of Figure 1, wherein a mechanism of wire release defines the radial expansion of the same surface;
Figure 5 is a perspective lateral view of the same endoprosthesis of Figure 4, with the mechanism of wire release that defines the radial expansion of the same surface;
Figure 6 is a front schematic view of a human body, wherein it is shown the insertion of the transfemoral endoprosthesis of the present invention;
Figure 7 is a front schematic view of a particular of a liver of a human body, with the point of connection of the upper hepatic veins to the inferior caval vein, wherein it is shown the installation of the endoprosthesis having the radial surface in the compressed position;
Figure 8 is a front schematic view of the same particular of a liver of Figure 7, wherein it is shown the installation of the endoprosthesis having the radial surface in the expanded position, so that the holes connecting the upper hepatic veins to the inferior caval vein are closed, keeping at the same time the circulation of blood inside;
Figure 9 is a lateral view of a particular of the front part of an endoprosthesis according to a second embodiment called *"by parachute"*, wherein a mechanism releasing the wires defines the radial expansion of the outer surface under action of the blood pressure;
Figure 10 is a lateral view of a particular of the front part surface of the same endoprosthesis of Figure 9, wherein a mechanism of wire traction defines the radial compression of the same surface;
Figure 11 is a perspective lateral view of a particular of the front part surface of the same endoprosthesis of Figure 10, wherein a mechanism of wire release defines the radial expansion of the same surface under the action of the blood pressure;
Figure 12 is a lateral view of a temporary venous transfemoral or trans-saphena endoprosthesis for a total vascular exclusion of the liver, according to the embodiment called *"by parachute"*;
Figure 13 is a perspective lateral view of the same endoprosthesis of Figure 12;
Figure 14 is a perspective lateral view of a temporary venous transfemoral or trans-saphena endoprosthesis for a total vascular exclusion of the liver, according to a third embodiment called "*by* flag", the third embodiment illustrating the scope of the present invention;
Figure 15 is a perspective lateral view of the same endoprosthesis of Figure 14, wherein the inner part is unrolled and defines the radial expansion of the outer surface;
It is here underlined that only few of the many conceivable embodiments of medical devices for total vascular exclusion of the liver are described, which are just some specific non-limiting examples, having the possibility to describe many other embodiments based on the disclosed technical solutions of the present invention, which is defined by appended claim 1.

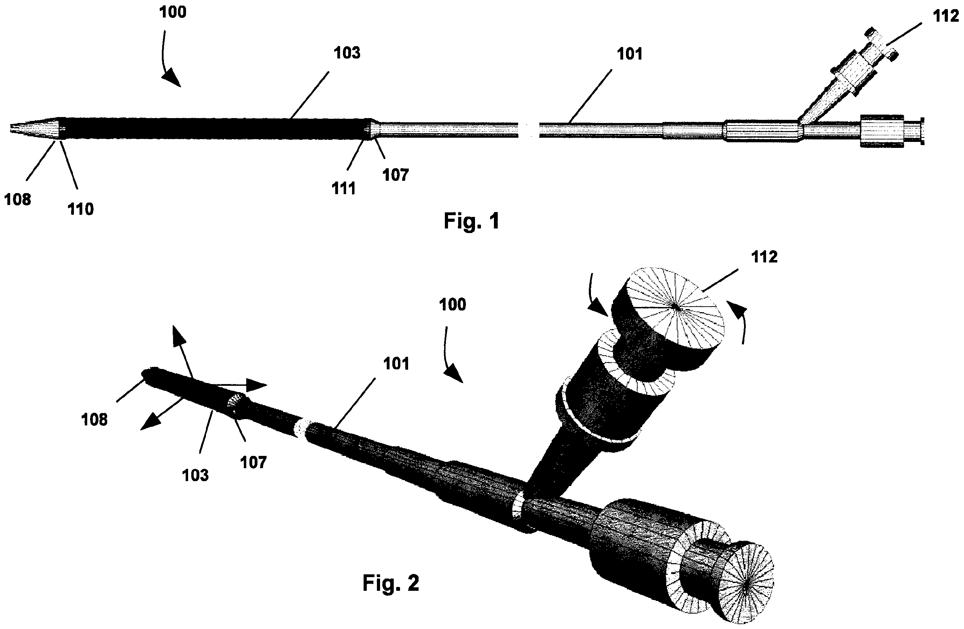

Figures 1 and 2 show a temporary venous transfemoral or trans-saphena endoprosthesis 100 for a total vascular exclusion of the liver 120. In particular, it can be used in most critical surgical operations involving this organ, like in example in *major hepatectomy* and in *hepatic trauma* with caval upper hepatic venous damage.

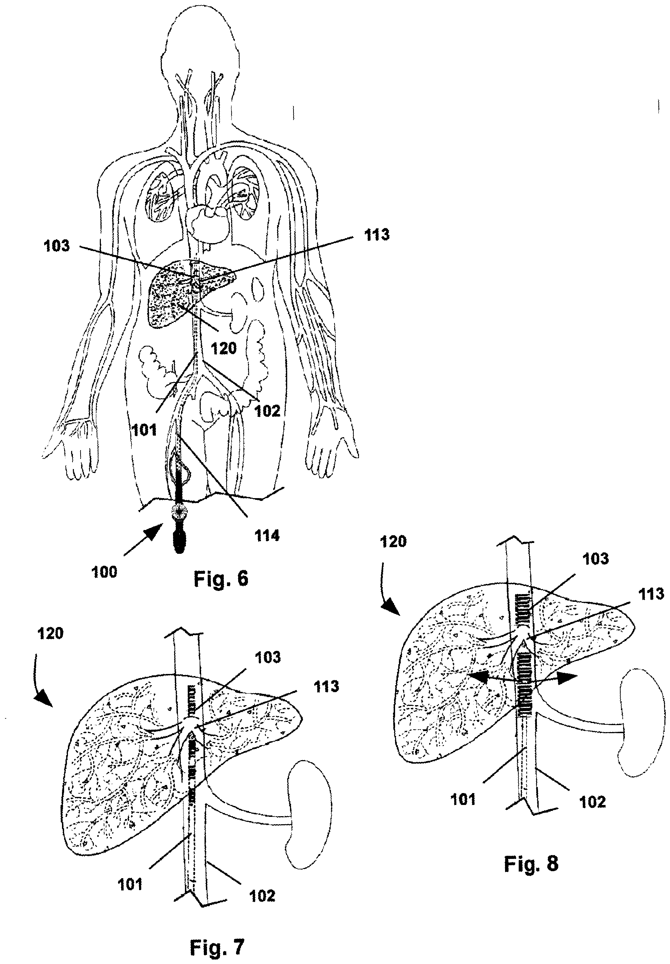

The endoprosthesis 100 comprises essentially an endovenous catheter 101, having the shape of a cylinder extended longitudinally, being flexible in the transverse direction, having a diameter in the order of the inner diameter of the same femoral vein or saphena vein 114, in order to be inserted from the same femoral vein or saphena vein 114, and being directed to the inferior caval vein 102, as shown in Figure 6. The endovenous catheter 101 further includes a cylindrical element 103 placed at the distal part of said endovenous catheter 101; the lower side of the cylindrical element 103 in the part close to said catheter 101 is oblique and has a rounded border.

The cylindrical element 103 includes a mechanism of radial expansion activated under control of a specific command device.

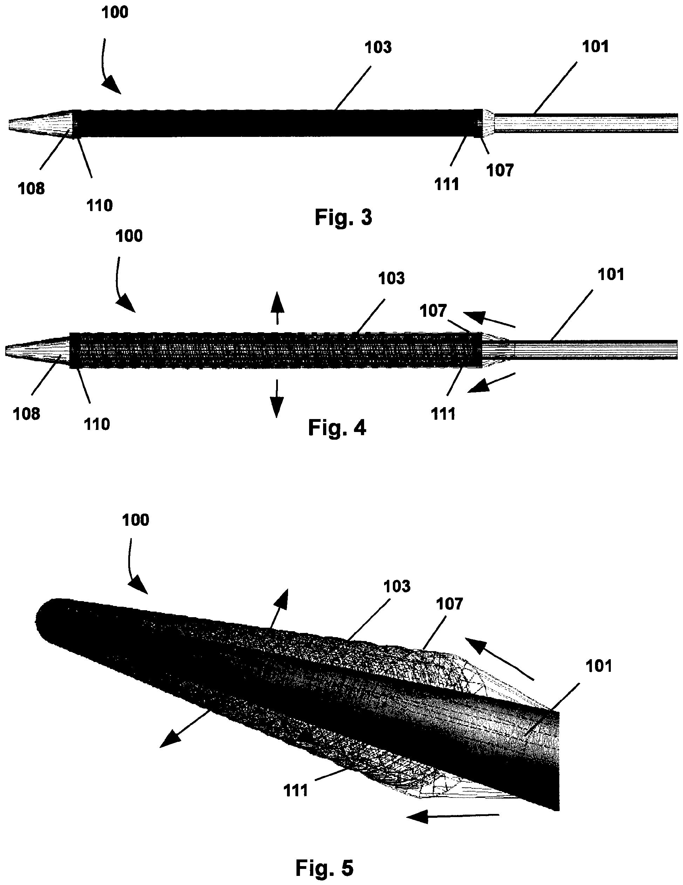

In such a way, under control of an operator, the endovenous catheter 101 is firstly installed by insertion from the femoral vein or saphena vein 114 directed to the caval vein 102, with the cylindrical element 103 placed in the caval tract of the upper hepatic veins, as shown in Figure 7. Then, said mechanism of radial expansion of said cylindrical element 103 is activated so that the lateral walls bond and close the holes connecting the upper hepatic veins 113 to the inferior caval vein 102 as shown in Figure 8. Therefore, the device permits the blood to flow inside the same cylindrical element 103 and prevents at the same time a return of blood to the liver 120. in such a way, with a simultaneous *Pringle maneuver* that stops the blood going to the liver 120, by blocking the hepatic peduncle (hepatic artery, common bile duct and portal vein) with a clamp or a tourniquet, a total vascular exclusion of the liver 120 is achieved. Figures 3, 4 and 5 show in detail the mechanism of radial expansion "*by expansion*" of said cylindrical element 103, according to a first embodiment of the invention.

The cylindrical element 103 is composed of a layer of flexible material, it has a lateral surface radially expandable and it is closed at both ends by a proximal conical element 107 and a distal conic element 108, with respective protective sealing caps 110 and 111. The lateral surface is compressed and housed by a structure of spiral rolled wires, each of them being connected to said proximal conical element 107.

The mechanism of radial expansion includes a releasing knob 112 directly connected to said wires, so that a rotary motion in one sense - clockwise or counter clockwise - causes the release of said wires, and therefore the enlargement "*by expansion*" of said radial surface, a rotary motion in the opposite sense - counter clockwise or clockwise - causes the traction of said wires, and therefore the compression of said radial surface.

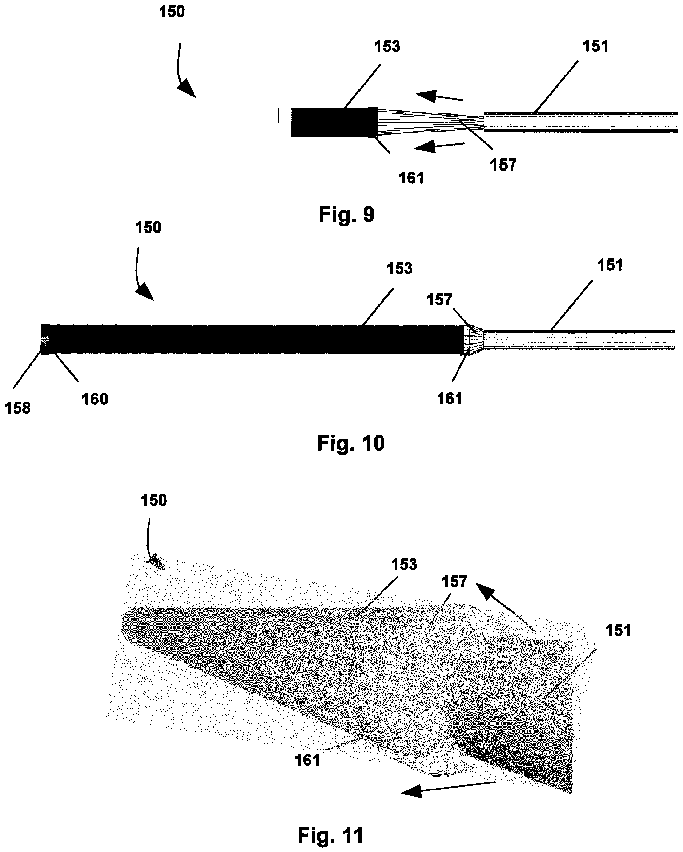

Figures 9, 10 and 11 show in detail the mechanism of radial expansion "*by parachute"* of said cylindrical element 103, according to a second embodiment. The cylindrical element 103 is composed of a layer of flexible material, it has a lateral surface radially expandable and it is closed at both ends by a proximal conical element 157 and a distal conic element 158, with respective protective sealing caps 160 and 161. The lateral surface is compressed and housed by a structure of spiral rolled wires, each of them being connected to said proximal conical element 157.

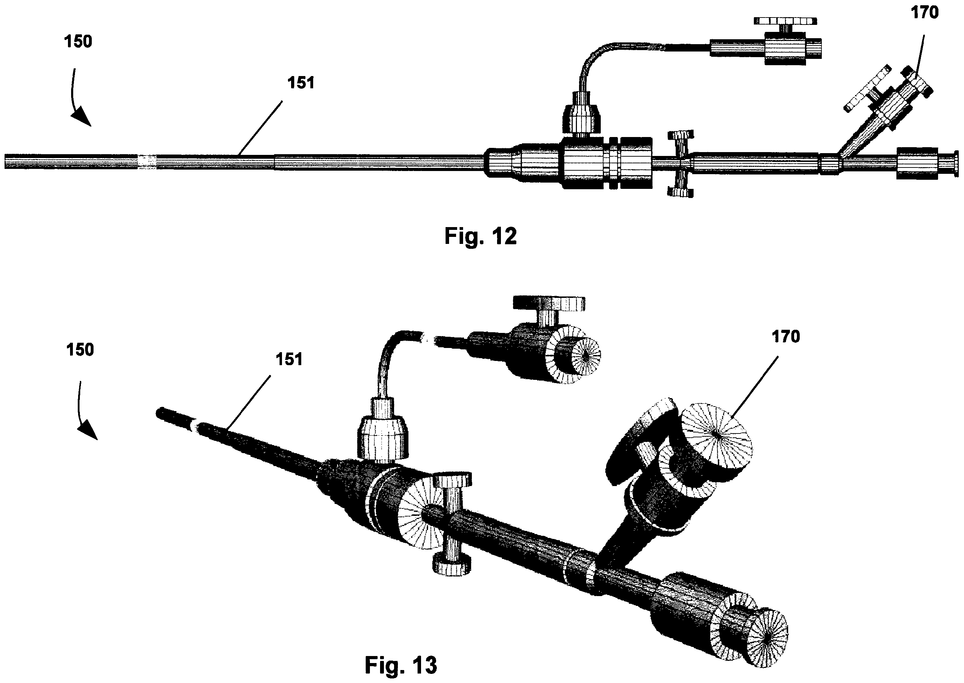

The mechanism of radial expansion of said cylindrical element 103 includes a piston device 170 applied to said endovenous catheter 150, as shown in Figures 12 and 13 and directly connected to said wires. In such a way, an activation of the device 170 causes the release of said wires, and therefore the enlargement *"by parachute"* of said radial surface under action of the blood pressure, and a disactivation of the device 170 causes the traction of said wires, and therefore the compression of said radial surface making it to re-enter into the endovenous catheter 150.

Figures 14 and 15 show in detail the mechanism of radial expansion *"by flag*" of said cylindrical element 103, according to a third embodiment, which illustrates the present invention. The cylindrical element 103 is composed of a layer of flexible material 131, preferably flat and having a triangular or squared shape, connected and rolled up on a supporting element 134 that is directed along the longitudinal axis of the endovenous catheter 130.

The supporting element 134 extends itself up to the proximal part 135 of the endovenous catheter 130, where it is connected to a control wheel 133. The supporting element 134, with the layer of material 131 rolled on it, is free to translate forward and backward inside a hollow cylindrical element 132.

The mechanism of radial expansion includes a piston device 170 that is activated by said control wheel 133: a rotary motion in one sense - clockwise or counter clockwise - causes the supporting element 134 to move forward outside of said hollow cylindrical element 132 with a progressive unrolling of said layer of material 131 and therefore the expansion of said radial surface; a rotary motion in the opposite sense - counter clockwise or clockwise - causes the supporting element 134 to move backward inside of said hollow cylindrical element 132 with a progressive rolling of said layer of material 131, and therefore the compression of said radial surface.

In order to provide the insertion and the installation of the endovenous catheter 101 in its final position inside the patient, it is possible to use the images coming from a CT-scan (*computed tomography*).

According to an embodiment of the invention, the cylindrical element 103 includes at its distal end a landmark composed of a radiopaque material.

In such a way, the same landmark results to be visible on images coming from a CT-scan, computed tomography, and said endovenous catheter 101 can be guided in real time to its final position in the hepatic tract of the inferior caval vein.

As an alternative, the outer surface of said endovenous catheter 101 presents a millimetric scale continuously visible to the operator of intervention.

In such a way, for a specific patient, starting from a CT-scan it is possible to extract the exact length of path that the endovenous catheter 101 should cover in order to reach the final position, and the millimetric scale permits to the operator to understand exactly at any time where it is placed the endovenous catheter 101 in respect to said final position.

The cylindrical element 103 can be composed of an elastic material having a memory of shape that, as a function of a specific temperature and/or another chemical-physical parameter, changes its geometrical shape until it reaches a specific previously defined shape, having a previously defined profile.

The cylindrical element 103 can be composed of an elastic material adapting itself perfectly to an irregular profile of the inner walls of the inferior caval vein 102, and to possible protrusions and concavities existing in the points of connection of the upper hepatic veins 113 in the same inferior caval vein 102.

The endovenous catheter 101 can be composed of modular and interchangeable parts having different sizes and extensions, with said cylindrical element 103 having different sizes and extensions, either in the initial position and in the expanded position.

In such a way, the endoprosthesis 100 can be adapted and results to be as the most appropriate as possible, in respect to a specific circulatory system and anatomy of the specific patient, and can be used as an endovenous bypass in the most effective, appropriate and safe possible way.

Therefore, the above third embodiment shows that the present invention achieves all the proposed objectives. In particular, it permits to obtain an endoprosthesis for a total vascular exclusion of the liver, overcoming all the drawbacks of the prior art, permitting a comfortable use because can be applied and adapted to different patients having different size and extension of the inner anatomical structures.

In particular, the endoprosthesis permits to keep the normal function of the caval vein to return the blood to heart, all during the time of the surgical intervention, avoiding a partial or total block of the circulation, and avoiding the possibility to a temporary extracorporeal circulation.

Furthermore, the present invention permits to achieve a highly precise control of the level of expansion of the prosthesis, a better perception by the surgeon of the reached level of expansion, a better adherence to the inner walls of the caval vein and an easier procedure if insertion and installation of the same prosthesis.

The present invention has been described for illustrative but not limitative purposes, according to its preferred embodiments, but it is clear that modifications and/or changes can be introduced by those skilled in the art without departing from the relevant scope, as defined in the enclosed claims.

## Claims

1. Temporary venous transfemoral or trans-saphena endoprosthesis (100) for a total vascular exclusion of the liver (120), to be used in most critical surgical operations like in example in *major hepatectomy* and in *hepatic trauma* with relevant venous damage, comprising:
- an endovenous catheter (101), with a millimetric scale, having the shape of a cylinder extended longitudinally, being flexible in order to be inserted from the femoral vein or saphena vein (114) directed to the inferior caval vein (102); the endovenous catheter (101) has a diameter in the order of the inner diameter of the same femoral veln or saphena vein (114);
- a cylindrical element (103) placed at the distal part of said endovenous catheter (101); the lower side of the cylindrical element (103) in the part close to said catheter (101) is oblique and has a rounded border;
- a mechanism of radial expansion of said cylindrical element (103) activated under control of a specific command device, so that, under control of an operator, the endovenous catheter (101) is firstly installed by insertion from the femoral vein or saphena vein (114) directed to the caval vein, with the cylindrical element (103) placed in the caval tract of the upper hepatic veins, and then said mechanism of radial expansion of said cylindrical element (103) is activated so that the lateral walls bond and close the holes connecting the upper hepatic veins (113) to the inferior caval vein (102), permitting the blood to flow inside the same cylindrical element (103) and preventing at the same time a return of blood to the liver (120); in such a way, with a simultaneous *Pringle maneuver* that stops the blood going to the liver (120), a total vascular exclusion of the liver (120) is achieved, wherein:
- said endovenous catheter comprises a hollow cylindrical element (132);
- said cylindrical element (103) is composed of a layer of flexible material (131), preferably flat and having a triangular or squared shape, connected and rolled up on a supporting element (134) that is directed along the longitudinal axis of the endovenous catheter (130); the supporting element (134) extends itself up to the proximal part (135) of the endovenous catheter (130), where it is connected to a control wheel (133); the supporting element (134), with the layer of material (131) rolled on it, is free to translate forward and backward inside said hollow cylindrical element (132);
- said mechanism of radial expansion of said cylindrical element (103) includes a piston device (170) that is activated by said control wheel (133): a rotary motion in one sense - clockwise or counter clockwise - causes the supporting element (134) to move forward outside of said hollow cylindrical element (132) with a progressive unrolling of said layer of material (131) and therefore the expansion of said radial surface; a rotary motion in the opposite sense - counter clockwise or clockwise - causes the supporting element (134) to move backward inside of said hollow cylindrical element (132) with a progressive rolling of said layer of material (131), and therefore the compression of said radial surface.

2. Temporary venous transfemoral or trans-saphena endoprosthesis (100) for a total vascular exclusion of the liver (120), according to previous claim 1, **characterized in that**:
- said cylindrical element (103) includes at its distal end a landmark composed of a radiopaque material,
so that the same landmark results to be visible on images coming from a CT-scan, computed tomography, and said endovenous catheter (101) can be guided in real time to its final position in the hepatic tract of the inferior caval vein.

3. Temporary venous transfemoral or trans-saphena endoprosthesis (100) for a total vascular exclusion of the liver (120), according to one or more of previous claims, **characterized in that**:
- the outer surface of said endovenous catheter (101) presents a millimetric scale continuously visible to the operator of intervention,
so that, for a specific patient, starting from a CT-scan it is possible to extract the exact length of path that the endovenous catheter (101) should cover in order to reach the final position, and the millimetric scale permits to the operator to understand exactly at any time where it is placed the endovenous catheter (101) in respect to said final position.

4. Temporary venous transfemoral or trans-saphena endoprosthesis (100) for a total vascular exclusion of the liver (120), according to one or more of previous claims, **characterized in that**:
- said cylindrical element (103) is composed of an elastic material having a memory of shape that, as a function of a specific temperature and/or another chemical-physical parameter, changes its geometrical shape until it reaches a specific previously defined shape, having a previously defined profile.

5. Temporary venous transfemoral or trans-saphena endoprosthesis (100) for a total vascular exclusion of the liver (120), according to one or more of previous claims, **characterized in that**:
- said cylindrical element (103) is composed of an elastic material adapting itself perfectly to an irregular profile of the inner walls of the inferior caval vein (102), and to possible protrusions and concavities existing in the points of connection of the upper hepatic veins (113) in the same inferior caval vein (102).

6. Temporary venous transfemoral or trans-saphena endoprosthesis (100) for a total vascular exclusion of the liver (120), according to one or more of previous claims, **characterized in that**:
- said endovenous catheter (101) is composed of modular and interchangeable parts having different sizes and extensions, with said cylindrical element (103) having different sizes and extensions, either in the initial position and in the expanded position,
so that the endoprosthesis (100) can be adapted and results to be as the most appropriate as possible, in respect to a specific circulatory system and anatomy of the specific patient, and can be used as an endovenous *bypass* in the most effective, appropriate and safe possible way.

## Patentansprüche

1. Transfemorale oder temporäre Trans-Saphenus-Endoprothese (100) für kompletten vaskulären Ausschluss der Leber (120), die bei den kritischsten chirurgischen Eingriffen verwendet wird, wie zum Beispiel bei größeren Hepatektomien mit verdächtiger suprahepatischer Kavalveneninfiltration und bei Lebertrauma mit großen vaskulären Läsionen, die folgendes umfasst:
- einen endovaskulären Katheter (101), mit einer zylindrischen, sich in Längsrichtung erstreckenden Form, der in der Querrichtung flexibel ist, um vorzugsweise von der Oberschenkel- oder Saphenusvene (114) direkt in die untere Hohlvene (102) eingefügt werden zu können und mit einem Durchmesser, der dem Innendurchmesser derselben Oberschenkel- oder Saphenusvene (114) entspricht;
- ein zylindrisches Element (103), das im distalen Teil des zuvor genannten endovaskulären Katheters (101) positioniert ist; der untere Rand des zylindrischen Elements (103), in der Nähe des proximalen Teils des Katheters (101), ist schräg mit abgeschrägter Ecke des distalen Teils;
- einen radialen Expansionsmechanismus zur Steuerung des zuvor genannten zylindrischen Elements (103), so dass, unter Kontrolle eines Operateurs, der endovaskuläre Katheter (101) zuerst eingebaut wird, indem dem man über die Oberschenkel- oder Saphenusvene (114) in die untere Hohlvene (102) gelangt, mit dem zylindrischen Element (103), das in Entsprechung des hinteren und suprahepatischen Kavalabschnitts positioniert ist und anschließend der zuvor genannte radiale Expansionsmechanismus des zylindrischen Elements (103) aktiviert wird, so dass die Seitenwände anhaften und die Eingangslöcher der suprahepatischen Venen (113) in der unteren Hohlvene (102) schließen und dem Blut ermöglichen, innerhalb desselben zylindrischen Elements (103) zu fließen und gleichzeitig eine Rückkehr des Bluts zur Leber (120) verhindert; auf diese Weise, mit einem gleichzeitigen Pringle-Manöver, dass den Blutzufluss zur Leber (120) sperrt, wird der kompletten vaskulären Ausschluss der Leber (120) bestimmt,
bei welchem der besagte endovaskuläre Katheter ein hohlzylindrisches Element (132) umfasst,
- das zuvor genannte zylindrische Element (103) besteht aus einer Schicht aus flexiblem Material (131), vorzugsweise eben und dreieckig oder rautenförmig, mit einem Stützelement (134) verbunden und spiralförmig umwickelt, das entlang der Längsachse des endovaskuläre Katheters (130) gerichtet ist; das Stützelement (134) erstreckt sich in Längsrichtung bis zum proximalen Teil (135) des Katheters (130), wo es an ein Steuerrädchen (133) angeschlossen ist; das Stützelement (134), mit einer Materialschicht (131), die um dieses gewickelt ist, ist frei, vorwärts und rückwärts zu laufen, innerhalb des besagten hohlzylindrischen Elements (132);
- besagter radialer Expansionsmechanismus zur Steuerung des zuvor genannten zylindrischen Elements (103) wird über das zuvor genannte Kontrollrädchen (133) aktiviert: eine Drehung in eine Richtung - im Uhrzeigersinn oder gegen den Uhrzeigersinn - bestimmt den Vorschub des Stützelements (134) außerhalb des Hohlzylinders (132), mit dem progressiven Abwickeln der Materialschicht (131), und anschließend die Kompression der radialen Oberfläche.

2. Transfemorale oder temporäre Trans-Saphenus-Endoprothese (100) für kompletten vaskulären Ausschluss der Leber (120), gemäß dem vorherigen Anspruch 1, ausgezeichnet durch die Tatsache, dass:
- das zylindrische Element (103) an seinem distalen Ende eine Schelle aus einem gegen elektromagnetische Strahlung undurchlässigem Material umfasst, so dass dieselbe Schelle in den Bildern einer CT (Computertomographie) sichtbar ist und der endovaskuläre Katheter (101) in Echtzeit in die Endposition in Entsprechung des hepatischen Abschnitts der unteren Hohlvene geführt werden kann.

3. Transfemorale oder temporäre Trans-Saphenus-Endoprothese (100) für kompletten vaskulären Ausschluss der Leber (120), gemäß einem oder mehreren der vorherigen Ansprüche, ausgezeichnet durch die Tatsache, dass:
- die äußere Oberfläche des endovaskulären Katheters (101) über eine graduierte Millimeterskala verfügt, die dem Operateur, der den Eingriff ausführt, konstant sichtbar ist, so dass es für einen speziellen Patienten möglich ist, aus einer CT (Computertomographie) die genaue Länge der Strecke, die der endovaskuläre Katheter (101) zurücklegen muss, um die Endposition zu erreichen, auszumachen und die graduierte Millimeterskala erlaubt dem Operateur, eine genaue Wahrnehmung, jederzeit, der Vorschubposition des endovaskulären Katheters (101) in Bezug auf diese Endposition zu haben.

4. Transfemorale oder temporäre Trans-Saphenus-Endoprothese (100) für kompletten vaskulären Ausschluss der Leber (120), gemäß einem oder mehreren der vorherigen Ansprüche, ausgezeichnet durch die Tatsache, dass:
- das zuvor genannte zylindrische Element (103) aus elastischem Memory-Material besteht, das in Entsprechung einer bestimmten Temperatur oder eines anderen chemisch-physikalischen Parameters seine geometrische Form verändert, bis zum Erreichen einer speziellen vordefinierten Form mit einem vordefinierten Längsprofil.

5. Transfemorale oder temporäre Trans-Saphenus-Endoprothese (100) für kompletten vaskulären Ausschluss der Leber (120), gemäß einem oder mehreren der vorherigen Ansprüche, ausgezeichnet durch die Tatsache, dass:
- das zuvor genannte zylindrische Element (103) aus elastischem Material besteht, das sich perfekt einem unregelmäßigen Profil der internen Wände der unteren Hohlvene (102) und den möglichen Unebenheiten und Konkavitäten in den Eingangspunkten der suprahepatischen Venen (113) in derselben unteren Hohlvene (102) anpasst.

6. Transfemorale oder temporäre Trans-Saphenus-Endoprothese (100) für kompletten vaskulären Ausschluss der Leber (120), gemäß einem oder mehreren der vorherigen Ansprüche, ausgezeichnet durch die Tatsache, dass:
- der zuvor genannte endovaskuläre Katheter (101) aus modularen und austauschbaren Teilen verschiedener Größe und Ausdehnung besteht, mit einem zylindrischen Element (103), austauschbar und in verschiedenen Größe und Ausdehnung, sowohl in der Ausgangsposition als auch in der ausgedehnten Position, so, dass die Endoprothese (100) angepasst ist und bestmöglich in Bezug auf das Kreislaufsystem und die Anatomie des jeweiligen Patienten geeignet ist und sie daher die endovaskuläre Bypass-Funktion auf effektivste, am besten geeignete und möglichst sichere Weise ausüben kann.

## Revendications

1. Endoprothèse transfémorale ou trans-saphène temporaire (100) pour l'exclusion vasculaire totale du foie (120), utilisée dans les opérations chirurgicales les plus critiques, comme par exemple lors d'hépatectomies majeures avec infiltration de la veine cave sus-hépatique présumée et lors de traumatismes hépatiques avec des lésions vasculaires majeures, comprenant :
- un cathéter endovasculaire (101), ayant une forme cylindrique étendue longitudinalement, étant souple dans la direction transversale pour pouvoir être inséré de préférence dans la veine fémorale ou saphène (114) vers la veine cave inférieure (102), et ayant un diamètre proportionné au diamètre intérieur de la susdite veine fémorale ou saphène (114) ;
- un élément cylindrique (103) positionné dans la partie distale dudit cathéter endovasculaire (101) ; la marge inférieure de l'élément cylindrique (103), à proximité de la partie proximale du cathéter (101), est oblique avec un coin arrondi, par rapport à l'autre extrémité de la partie distale ;
- un mécanisme d'expansion radiale de contrôle de l'élément cylindrique susdit (103), de sorte que, sous le contrôle d'un opérateur, le cathéter endovasculaire (101) soit dans un premier temps installé en entrant par la veine fémorale ou saphène (114) dans la veine cave inférieure (102), avec l'élément cylindrique (103) positionné en correspondance du trajet cave postérieur et sus-hépatique, et ensuite que le mécanisme susdit d'expansion radiale de l'élément cylindrique (103) soit activé, de sorte que les parois latérales adhèrent et ferment les trous d'insertion des veines sus-hépatiques (113) dans la veine cave inférieure (102), permettant au sang de circuler dans ledit élément cylindrique (103) et empêchant en même temps un retour de sang vers le foie (120) ; de cette façon, avec une manoeuvre de Pringle contextuelle, qui bloque le flux de sang vers le foie (120), on détermine l'exclusion vasculaire complète du foie (120),
où ledit cathéter endovasculaire comprend un élément cylindrique creux (132),
- le susdit élément cylindrique (103) est constitué d'une couche de matériau souple (131), de préférence plat et de forme triangulaire ou en forme de losange, connexe et enroulé en spirale sur un élément de support (134) qui est dirigé le long de l'axe longitudinal du cathéter endovasculaire (130) ; l'élément de support (134) s'étend en longueur jusqu'à la partie proximale (135) du cathéter (130), où il est lié à une roulette de contrôle (133) ; l'élément de support (134), avec la couche de matériau (131) enroulé sur celui-ci, est libre de glisser en avant et en arrière, à l'intérieur dudit élément cylindrique creux (132) ;
- ce mécanisme d'expansion radiale de contrôle, du susdit élément cylindrique (103), est activé par la roulette de contrôle susdite (133) : une rotation dans un sens - horaire ou antihoraire - détermine l'avancement de l'élément de support (134) hors du cylindre creux (132), avec le déroulement progressif de la couche de matériau (131), et donc la dilatation de la surface radiale, une rotation dans le sens inverse - antihoraire ou horaire - détermine le recul de l'élément de support (134) à l'intérieur du cylindre creux (132) avec l'enroulement progressif de la couche de matériau (131), et donc la compression de la surface radiale.

2. Endoprothèse transfémorale ou trans-saphène temporaire (100) pour l'exclusion vasculaire totale du foie (200), selon la revendication 1 précédente, **caractérisée par le fait que** :
- l'élément cylindrique susdit (103) comprend à la propre extrémité distale un collier constitué de matériau mat à la radiation électromagnétique,
de sorte que le collier susdit soit visible sur les images provenant d'une TDM (tomodensitométrie) et le cathéter endovasculaire (101) puisse être guidé en temps réel dans la position finale en correspondance du trajet hépatique de la veine cave inférieure.

3. Endoprothèse transfémorale ou trans-saphène temporaire (100) pour l'exclusion vasculaire totale du foie (200), selon une ou plusieurs revendications précédentes, **caractérisée par le fait que** :
- la surface extérieure du cathéter endovasculaire (101) présente une règle graduée en millimètre visible en permanence par l'opérateur qui exécute l'intervention,
de sorte que, pour un patient spécifique, il soit possible d'identifier à partir d'une TDM (tomodensitométrie) la longueur exacte du parcours que le cathéter endovasculaire (101) doit parcourir pour atteindre la position finale, et que la règle graduée en millimètre permette à l'opérateur d'avoir une perception exacte, à tout moment, de la position d'avancement du cathéter endovasculaire (101) par rapport à cette position finale.

4. Endoprothèse transfémorale ou trans-saphène temporaire (100) pour l'exclusion vasculaire totale du foie (200), selon une ou plusieurs revendications précédentes, **caractérisée par le fait que** :
- l'élément cylindrique susdit (103) est constitué d'un matériau élastique à mémoire de forme qui, en correspondance d'une température déterminée ou d'un autre paramètre chimique et physique, modifie la propre forme géométrique jusqu'à une forme spécifique prédéfinie, ayant un profil longitudinal prédéfini.

5. Endoprothèse transfémorale ou trans-saphène temporaire (100) pour l'exclusion vasculaire totale du foie (200), selon une ou plusieurs revendications précédentes, **caractérisée par le fait que** :
- l'élément cylindrique susdit (103) est constitué d'un matériau élastique s'adaptant parfaitement à un profil irrégulier des parois intérieures de la veine cave inférieure (102) et aux éventuelles aspérités et concavités aux points d'insertion des veines sus-hépatiques (113) dans la même veine cave inférieure (102).

6. Endoprothèse transfémorale ou trans-saphène temporaire (100) pour l'exclusion vasculaire totale du foie (200), selon une ou plusieurs revendications précédentes, **caractérisée par le fait que** :
- le cathéter endovasculaire susdit (101) est constitué de parties modulaires et interchangeables de différentes grandeurs et extensions, avec un élément cylindrique (103) interchangeable et de différentes grandeurs et extensions, aussi bien dans la position initiale que dans la position étendue, de sorte que l'endoprothèse (100) soit adaptée et soit la plus appropriée possible, par rapport au système circulatoire et à l'anatomie spécifique du patient, et puisse donc exercer la fonction de by-pass intraveineux de la façon la plus efficace, appropriée et sûre possible.
